# EUROPEAN PATENT SPECIFICATION

(11) **EP 2 695 622 B1**
(45) Date of publication and mention of the grant of the patent: **26.12.2018**
(21) Application number: 12767681.5
(22) Date of filing: 17.01.2012
(51) Int. Cl.: A61L 15/28, A61F 13/02

(54) **A CHITOSAN WOUND DRESSING AND ITS METHOD OF MANUFACTURING**
CHITOSANWUNDVERBAND UND VERFAHREN ZU SEINER HERSTELLUNG
PANSEMENT POUR PLAIES À BASE DE CHITOSANE ET SON PROCÉDÉ DE FABRICATION

(30) Priority: 02.04.2011 CN 201110082863
(43) Date of publication of application: 12.02.2014
(73) Proprietor: Foshan United Medical Technologies Ltd, Guangdong 528225 (CN)
(72) Inventor: MO, Xiaohui, Foshan Guangdong 528225 (CN); WANG, Xiaodong, Foshan Guangdong 528225 (CN); WU, Zhenjun, Foshan Guangdong 528225 (CN)
(74) Representative: Wilson Gunn
(86) International application number: PCT/CN2012/070441
(87) International publication number: WO 2012/136082

(56) References cited:
- WO-A1-97/03708
- WO-A1-2008/038864
- WO-A1-2008/072230
- CN-A- 1 833 731
- CN-A- 101 235 100
- YIMIN QIN: "The preparation and characterization of chitosan wound dressings with different degrees of acetylation", JOURNAL OF APPLIED POLYMER SCIENCE, vol. 107, 2008, pages 993-998, XP002730674,
- DATABASE CA [Online] CHEMICAL ABSTRACTS SERVICE, COLUMBUS, OHIO, US; ZHU, CHANGJUN ET AL: "Effect of acetylation on the properties of chitosan wound dressings", XP002730675, retrieved from STN Database accession no. 2008:766038 & ZHU, CHANGJUN ET AL: "Effect of acetylation on the properties of chitosan wound dressings", FANGZHI XUEBAO , 29(4), 18-21 CODEN: FCHPDI; ISSN: 0253-9721, 2008,
- LIHONG FAN ET AL.: "Preparation of alginate/half-acetylation blend fibers", POLYMER PREPRINTS, vol. 2005, no. 46(1), 2005, page 304, XP002730676,
- LI, JUNFENG ET AL.: 'Research on Preparation of Water-soluble Chitosan by N-acetylating' GUANGZHOU CHEMICAL INDUSTRY vol. 38, no. 7, 2010, pages 115 - 117, XP008170755

## Description

### FIELD OF THE INVENTION

The invention relates to methods of manufacturing acylated chitosan wound dressings which have a high absorption capacity and a high wet strength. The dressing forms a clear gel on contact with distilled water and aqueous solutions such as saline and solution A (a solution containing 8.298g/l of sodium chloride and 0.368 g/l of calcium chloride dehydrate and distilled water).

### BACKGROUND OF THE INVENTION

Chitin widely exists in nature. It is a major component of the fungal cell wall and the carapaces of shrimps, crabs and insects. Also, chitin has the largest amount of nitrogen groups, only second to proteins amongst all natural polymers in the world,

Chitosan, a polyglucosamine, (1-4)-2-amino-2-deoxy-β-D-glucose, is a product of deacetylation of chitin with concentrated alkaline solution. It is a widely existing polysaccharide and has many advantages, such as being non-toxic, non-irritative, biocompatible and having no immunogenicity. It also has other properties that make chitosan an ideal material for a wound dressing such as being biodegradable, haemostatic, bacteriostatic etc.

It is well known that chitosan has a good haemostatic property by adhering its molecule to red blood cells to stop bleeding. Chitosan is the only natural polysaccharide with positive charge existing on the earth; it can adhere to the surface of red blood cells, which are negatively charged. This adhesion promotes blood coagulation, thereby causing stanch. Moreover the molecule of chitosan can polymerize with the blood and form a reticular structure, which further captures red blood cells.

There is a large quantity of free amino groups in the molecule of chitosan that affects the existence of the carboxyl group of the protein on the cell surface. The existence of the free amino group is the reason for the antimicrobial property of chitosan. On one hand the free amino group causes the accumulation of bacteria cytoplasm. Chitosan can penetrate into the cells and attach to the cytoplasm which contains anions, thus disturbing the cell metabolism and causing the death of bacteria. Another explanation of chitosan's bacteriostatic properties is that, the positive charge of a chitosan molecule neutralizes the negative charge of a bacterium's cell membrane causing damage to the bacterium cell wall, thus allowing small molecules of chitosan to penetrate through the damaged cell membrane into the cell nucleus. In the cell nucleus, chitosan binds to DNA molecules and inhibits the formation of bacterial DNA, giving antimicrobial capability.

However, chitosan is, by the presence of these free amino groups, a cationic polymer, which causes the material to be insoluble in an alkaline medium and water. It is only soluble in some acids. This makes chitosan very low in absorption capacity. The degree of deacetylation of chitosan affects its solubility in water. The material becomes water soluble only when the degree of deacetylation is equal to 50%. This restricts the use of the chitosan. Consequently, there is an abundance of research into the improvement of the absorption capacity of the chitosan material, those improvements exist in using powder, films, non-woven fabrics, tapes, bandages, solutions, hydrogels, cotton tissue, lotions and creams.

Chinese patent application CN1715465A disclosed a high absorbent carboxymethyl chitosan, which is obtained by reaction of chitosan fibre with halogenated acetic acid. This carboxymethyl chitosan has a degree of carboxymethyl substitution between 0.01 and 0.50. It possesses high absorption capacity and is suitable for making wound dressings, especially for bleeding wounds and highly exudating wounds. However, this method uses hazardous materials such as halogenated acetic acid, which is toxic, and an inorganic alkali, which is caustic.

US patent application US2008/0241229A1 and Chinese patent application CN1833732A disclosed an antimicrobial carboxymethyl chitosan wound dressing. According to said patent applications, chitosan fibre is etherified before being subjected to plasma treatment. Said wound dressing swells and forms an elastic gel when in contact with water. It can be used in surgical wounds, burns and other chronic wounds. But the plasma treatment of this invention is very expensive.

Chinese patent application CN1500525A disclosed a pre-cation chitin derivative wound dressing which is water-soluble. The chitin derivative has a degree of deacetylation of greater than 50% and a degree of substitution of less than 0.3. Additionally, the wound dressing contains antimicrobial drugs which can promote epidermal growth and tissue recovery. The wound dressing is in the form of spray and film and can be used in surgical wounds, burns, trauma, ulcers and other chronic wounds. But the addition of antimicrobial drugs will increase the cost of manufacture.

Among numerous chitosan derivatives, carboxymethyl chitosan has been widely studied and reported. However, besides the carboxymethyl group, the introduction of a carboxyl group or an amino group into the hydroxyl group has the same effect on improving the absorption capacity of the chitosan material. A typical example is acylated chitosan which can be formed by treating chitosan with an anhydride in the acidic medium. When in contact with blood, acylated chitosan rapidly absorbs the moisture in the blood, increasing the blood viscosity and facilitates blood coagulation. Acylated chitosan containing a carboxyl group has an excellent absorption capacity. It is a potential medical absorbent material.

European patent EP 1487507B1 disclosed a wound dressing which absorbs water or wound fluid and then swells or forms a gelatinous mass. It is prepared by a reaction of chitosan or chitin with a cyclic anhydride, such as cis-aconitic acid. The said material consists of a chemically modified chitosan, wherein at least one saccharide residue has grafted onto a side chain comprising of two or more carboxylate groups wherein the side chain has only one linkage to chitosan. This wound dressing is in the form of beads, flakes, powder, a film, a fibrous pad, a web, a woven or non-woven fabric, a freeze-dried sponge or a foam and can be used as a dressing for chronic wounds. The disadvantage of this method is that the reaction process is very complex.

US patent application US2009/0035356A1 described a chitosan hemostat produced by the reaction of chitosan with maleic anhydride derivative. This product has the property of being haemostatic. But the reaction also produces maleic anhydride derivative simultaneously, increasing the reaction time and cost.

US patent US4031025 disclosed a modified chitosan product as a skin moisturizer in a cosmetic agent composition. The product has good moisture retention capacity. The patent also describes a method of acylation of chitosan with a diacid anhydride. However, this product is used as a material for cosmetic application, not being suitable for wound dressings.

Chinese patent application CN101624778A disclosed a method of producing chitosan fibre with high water absorbency. Chitosan fibre is reacted with succinic anhydride to form acylated chitosan fibre, which swells and becomes gel when in contact with aqueous solution. But this patent only explains the method and the principle of the reaction, without further exploring the application of this material and defining the parameters for wound care application.

Some of the above disclosures have employed the acylation process to chemically modify the chitosan material. But their processes are very complicated and use a lot of chemicals. Importantly, most of them use chitosan powder as the raw material, resulting in powderous acylated chitosan. Unlike soft fibrous chitosan material, the acylated chitosan powder is difficult to use on chronic wounds.

European patent EP0616650B1 described a carboxymethyl solvent spun cellulose fibre (Lyocell) that has a good water absorption capacity. From this EP0680344B1 introduced a wound dressing using the above material. The latter explained procedures to make a wound dressing from the carboxymethyl solvent spun cellulose fibre and gave parameters of such a process. The dressing made from this material is an absorbent gel forming wound dressing. However, this material is neither bacteriostatic nor haemostatic. Also it has a low wet strength, making it difficult to remove from the wound.

Journal of Applied Polymer Science, vol.107, 993-998 (2008) discloses the preparation of chitosan wound dressings with different degrees of acetylation. The acetylation is carried out with acetic anhydride in methanol.

In general, a chronic wound produces about 50 ml of exudate over a 24 hour period. The absorption capacity of ordinary gauze (weighs about 1 gram) is only at the level of 200%, absorbing 2 grams of the exudate. This is insufficient for the care of chronic wounds. Moreover, most of this type of chronic wound is infected by bacteria, therefore it would be ideal if a wound dressing can also be antimicrobial or bacteriostatic. Also some wounds bleed therefore a wound dressing with haemostatic property is advantageous.

With such a high absorption capacity, a wound dressing will become very heavy upon absorbing wound exudates. Ordinary wound dressings such as carboxymethyl solvent spun cellulose wound dressings have a low wet strength and can therefore break on removal. Therefore, the wet strength has become an important consideration when it comes to selecting a wound dressing. Additionally, reports suggest that MMP exists at some stage of a wound healing which could slow the healing process. Chitosan has the ability to inhibit the production of MMPs.

Internationally, Solution A has become a standard testing solution for dressing absorbency and wet strength. The solution A contains calcium and sodium ions that are similar to that in the wound exudates. Solution A contains 8.298 g/l of sodium chloride, 0.368 gram of calcium chloride dehydrate and distilled water.

Therefore an object of this invention is to provide a safe and low cost method for the manufacturing of a wound dressing for the application on chronic wounds such as ulcers and burns that utilises the properties of being absorbent, bacteriostatic and haemostatic of the acylated chitosan fibre.

According to the present invention, there is provided a method for the preparation of a wound dressing comprising acylated chitosan fibre in which the acylated chitosan fibre has a degree of substitution of 0.10-0.50 and wherein the dressing has a minimum wet strength of 0.3 N/cm, the method comprising:
preparing the acylated chitosan fibre by reacting chitosan fibre with an anhydride in a solvent; and making an acylated chitosan dressing through a weaving, knitting or nonwoven process, and then through cutting, packing and sterilizing processes.;
or preparing a standard chitosan fabric through a textile process such as weaving, knitting or nonwoven; and chemically modifying the said fabric through an acylation process comprising reacting the chitosan fibre with an anhydride in a solvent, and then through cutting, packing and sterilizing processes,
wherein the anhydride is selected from succinic anhydride, glutaric anhydride, maleic anhydride, phthalic anhydride and diglycolic anhydride, and wherein the solvent is ethanol.

In comparison with un-treated chitosan material, acylated chitosan fibre prepared by methods of the invention is highly absorbent while maintaining some of the properties of chitosan fibre such as being haemostatic and bacteriostatic. The high absorption capacity of the acylated chitosan fibre is due to the introduction of carboxyl group which is hydrophilic. Through the control of the degree of substitution of the acylation process, the fibre and the dressing can be made with high absorbency as well as high wet strength. The dressing also forms a clear gel on contact with distilled water and aqueous solutions such as, saline and solution A. Importantly, the acylated chitosan wound dressing remains intact on absorbing wound exudates, making the dressing changes easier.

In contrast, the traditional gauze has very low absorbency, it often adheres to the wound and becomes difficult to remove, causing patient discomfort.

The wound dressing prepared by the methods of this invention has a high wet strength. The wet strength here refers to the maximum breaking force of a wound dressing when it is fully absorbent with Solution A. The wet strength normally includes wet strength in machine direction and in cross machine direction of a dressing. The wet strength referred to in this invention is the average wet strength of that in machine direction and that in cross machine direction.

The wet strength of a dressing can be obtained by using an Instron or a similar tensiometer to test its strength, when in wet, in machine direction and cross machine direction separately. The dressing's wet strength is the average of both strengths. The dressing remains intact on absorbing wound exudates and does not adhere to the wound.

The degree of substitution (DS) of the acylation process of this invention is 0.1-0.5, preferably 0.2-0.4. The degree of substitution of the acylation refers to the number of amino group and hydroxyl group that have been acylated in the acylation process per glucose molecule. It has been found that the dressing has a good balance of absorbency and wet strength when DS is controlled between 0.1 and 0.5. The absorbency and wet strength of the dressing becomes even higher and more consistent if the DS is controlled to between 0.20 and 0.40. This way the dressing can be removed as one piece after absorbing wound exudates.

The dressing prepared by method of this invention has haemostatic properties. This can be measured by using a Spectrophotometer to determine the coagulation time of a sample of rabbit blood. Blood containing calcium chloride is covered with an acylated chitosan dressing and the OD is obtained at the weave length of 540 nm. From comparing OD, a coagulation time of 120 seconds is obtained.

The wound dressing prepared by method of this invention has bacteriostatic properties. This can be observed from the following experiment: 1) placing an acylated chitosan wound dressing onto a Petri-dish coated with a bacterium such as Escherichia coli or Staphylococcus aureus or Bacillus subtilis, and 2) observing the growth after 24 hours. It can be found that the area underneath of the dressing does not have growth while other areas have clear signs of bacteria growth.

This invention uses chitosan fibre as the raw material, through an acylation process which employs specific anhydrides as the reagent, ethanol as the solvent. There are large amounts of amino groups and hydroxyl groups within the structure of the chitosan fibre, the process of acylation occurs with these groups. Also the existence of the amino group makes the chitosan unique in terms of biological functions and ease of chemical modifications.

The chitosan fibre used in this invention has a degree of deacetylation of above 50%, preferably above 70%, most preferably 80% or above. The deacetylation of a chitosan fibre refers to the percentage of the acetyl group that has been removed from the molecular chain. In general chitosan that has certain industrial value shall have deacetylation of 70% or above.

The anhydride used in the fibre chemical modification of the chitosan fibre is selected from saturated or unsaturated organic diacid anhydrides. The diacid anhydride is selected from succinic anhydride, glutaric anhydride, maleic anhydride, phthalic anhydride, diglycolic anhydride. The diacid anhydride is preferably succinic anhydride. The chemical modification process can be illustrated as follow:

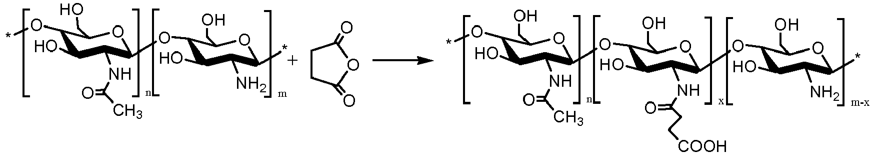

By controlling the molar ratio between anhydride and amino group of chitosan fibre, it is possible to obtain acylated chitosan fibre with different degrees of substitution, which determines the gelling characteristics of the fibre. The molar ratio for the reaction shall be equal to or greater than 1:1, preferably 2:1, most preferably 4:1.

The acylation process of this invention involves heating the chitosan fibre (with a degree of deacetylation of above 50%) in an ethanol solution containing anhydride at a concentration of 0.1 g/ml - 0.2 g/ml to a temperature of 30-100 °C, preferably 50-70 °C for about 10-120 minutes, preferably 30-90 minutes.

On completion of the reaction, the fibre is washed with ethanol to remove the residue from the reaction, followed with a drying process to obtain the acylated chitosan fibre.

The chitosan and acylated chitosan of this invention is staple fibre. The fibre length shall be controlled between 3mm and 125 mm, preferably between 20mm and 85 mm.

Preferably the linear density of the acylated chitosan fibre shall be between 0.5 dtex and 5 dtex.

To assist the process of manufacturing the dressing after the acylation process, i.e. to improve the fibre softness, surface friction or electrostatic properties, spin finish or lubricant are applied to the fibre surface at a concentration of between 0.05% and 5.5%, preferably between 0.5% and 2%. The typical spin finish includes Tween 20, Tween 60, Tween 80, lubricant 602 etc.

To further improve the antimicrobial properties of the acylatyed fibre, antimicrobial agents can be added to the fibre structure or onto the fibre surface. For example, silver or PHMB solutions can be sprayed onto the fibre surface at a concentration of 0.05%-20%, preferably 0.1-10%, based on the finished fibre weight. Additionally silver or silver compounds, such as nano silver, Alphasan etc, can be added to the fibre structure during the fibre manufacturing process.

In one example of this invention, some untreated chitosan fibres were blended with acylated chitosan fibre during the dressing manufacturing stage (i.e. carding). This way, the dressing's wet strength can be further enhanced whilst maintaining its antimicrobial property.

In another example, the acylated chitosan wound dressing contains some calcium alginate fibre so that the dressing has an ability to donate calcium.

In another example, some chemically modified cellulose fibres were blended with the acylated chitosan fibre during the dressing manufacturing process such as carding. The addition of the chemically modified cellulose fibre increases the dressing absorption capacity. Preferably the chemically modified cellulose fibre is carboxymethyl solvent spun cellulose fibre.

To further improve the dressing's wet strength, a small percentage of standard textile fibre such as viscose, polyester or nylon can be blended with acylated chitosan fibre. Those textile fibres can reinforce the dressing and increase the dressing's wet strength significantly.

The wound dressing prepared by methods of this invention can be in the form of woven, knitted or nonwoven fabrics, and can be manufactured through one of the following methods:
A. Fibre Modification option:
   1) Chemically modifying the chitosan fibre into acylated chitosan fibre;
   2) Converting the acylated chitosan fibre through weaving, knitting or via nonwoven processing into fabrics;
   3) Cutting the fabric into dressing, then packing and sterilising the product to make it suitable for wound care application.
B. Fabric Modification option:
   1) Converting the standard chitosan fibre through weaving, knitting or via nonwoven processing into fabrics;
   2) Chemically modifying the chitosan fabric into acylated chitosan fabric;
   3) Cutting the acylated chitosan fabric into dressing, then packing and sterilising the product to make it suitable for wound care application.

The wound dressing prepared by methods of this invention has a high absorption capacity and can provide a moist environment for the wound. It can also maintain a high wet strength whilst absorbing wound exudate, allowing an intact removal of the dressing, preventing damage of the newly formed tissues and causing less patient discomfort during the dressing change. Therefore the said acylated chitosan wound dressing can be used in the care of chronic wounds.

Additionally Chitosan fibre has the properties of being haemostatic, bacteriostatic and an inhibitor of MMP. These properties can prevent infection from microorganisms, providing a favorable environment for wound healing, therefore making the acylated chitosan wound dressing suitable for the care of chronic wounds.

In order that the invention may be more clearly understood embodiments thereof will now be described, by way of example, with reference to the accompanying drawings of which:
Fig. 1 shows bacterial growth of Staphylococcus aureus in a petri-dish containing a wound dressing prepared according to the present invention;
Fig. 2 shows bacterial growth of Bacillus subtilis in a petri-dish containing a wound dressing prepared according to the present invention;
Fig. 3 shows bacterial growth of Pseudomonas aeruginosa bacilli in a petri-dish containing a wound dressing prepared according to the present invention;
Fig. 4 shows bacterial growth of Escherichia coli in a petri-dish containing a wound dressing prepared according to the present invention; and
Fig. 5 shows the zone of inhibition of a wound dressing prepared according to the present invention in a petri-dish.

### DETAILED DESCRIPTION OF THE INVENTION

### Example 1

An absorbent acylated chitosan fibrous wound dressing can be made by following the below steps:
1. Preparation of the reaction solution with a concentration of 0.1g/ml of succinic anhydride: weigh 894g of succinic anhydride and dissolve it in 8940 ml of ethanol, keep stirring until all succinic anhydride is fully dissolved.
2. Weigh 400g of chitosan fibre (degree of deacetylation above 90%, fibre linear density 1.7 dtex). This will give a molar ratio of anhydride and amino group of chitosan fibre of 4:1. Submerse the fibre in ethanol solution for 30 minutes, then squeeze off all liquid.
3. Place the chitosan fibre into the reaction solution prepared earlier, heat the solution to 70°C for 40 minutes.
4. Stop the reaction and take the fibre out of solution, squeeze all liquid out of the fibre, then wash the fibre with ethanol to remove residue.
5. Place the washed fibre in an ethanol solution containing Tween 20 for 30 minutes, then squeeze off all liquids, dry the fibre before crimping and cutting the fibre into staple fibre (length 51 mm).
6. Measure the degree of substitution of the treated fibre, DS=0.35.
7. Place the fibre through a carding and nonwoven line and convert the fibre into a nonwoven felt with a base weight of 120g/m², cut the felt into 5x5 cm or 10x10 cm dressings and pack them into pouches before putting the packed dressing through a gamma radiation process for sterilisation.

### Example 2

Measurement of the absorption capacity of the acylated chitosan wound dressing (following the method of BS EN 13726-2002).
1. Weigh a 5x5 cm acylated chitosan wound dressing made from example 1, W1=0.3557g
2. Place the dressing into a Petri-dish containing solution A. The volume of the solution A is 40 times of the weight of the dressing.
3. Place the Petri-dish in an oven for 30 minutes at 37°C.
4. Lift the sample and hold it still for 30 seconds, weigh the mass of the dressing W2=6.6330 g.
5. The absorption capacity of the dressing is A=(W2-W1)/W1=17.65 g/g.

### Example 3

Measurement of the wet strength of the acylated chitosan wound dressing:
Take a 10x10 cm dressing made from example 1, cut a 2x10 cm strip from the dressing, then cut another 2x8 cm strip from the dressing at 90 degrees from the previous strip. These two strips are samples for wet strength for the machine direction and cross machine direction.

Place both strips into a Petri-dish containing solution A for 30 seconds, remove the excess solution and test the maximum breaking force of each strip using a tensiometer. This obtains the wet strength of the dressing in machine direction 1.04N/cm, the cross machine direction 1.52N/cm, the average wet strength of 1.28 N/cm.

### Example 4

Measurement of the bacteriostatic property of the acylated chitosan wound dressing made from example 1:
1. Cut the dressing made from example 1 into a 2x2 cm square.
2. Place the sample onto a Petri-dish that has been covered with 0.25 ml of 10⁶-10⁷ cfu/g Staphylococcus aureus.
3. Place the Petri-dish into an oven at a temperature of 37°C and observe the growth of the bacteria on the plate.

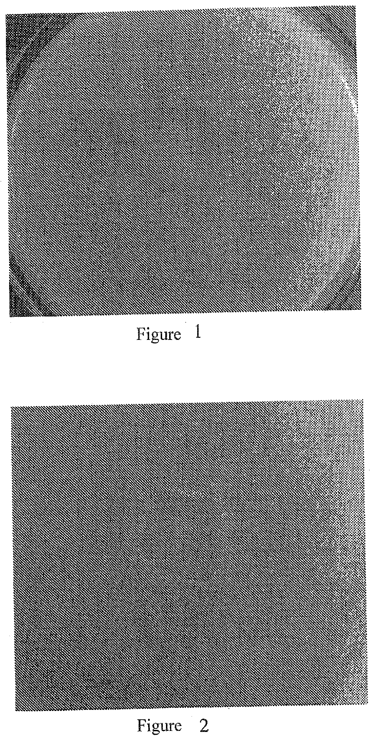

It can be seen that the area underneath the dressing does not show signs of bacterial growth whilst other areas display significant growth of the bacteria, as shown in Fig. 1.

### Example 5

Another method of making an absorbent acylated chitosan fibrous wound dressing:
1. Preparation of the reaction solution with a concentration of 0.05 g/ml of succinic anhydride: weigh 1029 g of succinic anhydride and dissolve it in 20.58 L of ethanol, keep stirring until all succinic anhydride is fully dissolved.
2. Weigh 650 g of chitosan fibre (degree of deacetylation of 85%, fibre linear density was 1.7 dtex). This will give a molar ratio of anhydride and amino group of chitosan fibre of 3:1. Submerse the fibre in ethanol solution for 30 minutes, and then squeeze off all liquid.
3. Place the chitosan fibre into the reaction solution prepared earlier, heat the solution to 60 °C for 75 minutes.
4. Stop the reaction and take the fibre out of solution, squeeze all liquid off the fibre, then wash the fibre with ethanol to remove residue.
5. Place the washed fibre in an ethanol solution containing Tween 20 for 30 minutes, then squeeze off all liquids, dry the fibre before crimping and cutting the fibre into staple fibre (length 60 mm).
6. Measure the degree of substitution of the treated fibre, DS=0.33.
7. Place the fibre through a carding and nonwoven line and convert the fibre into a nonwoven felt with a base weight of 160g/m², cut the felt into 5x5 cm or 10x10 cm dressings and pack them into pouches before putting the packed dressing through an EtO (Ethylene Oxide) process for sterilisation.

### Example 6

Measurement of the absorption capacity of the acylated chitosan wound dressing (following the method of BS EN 13726-2002).
1. Weigh a 5x5 cm acylated chitosan wound dressing made from the example 5, W1=0.4070g
2. Place the dressing into a Petri-dish containing solution A. The volume of the solution A is 40 times of the weight of the dressing.
3. Place the Petri-dish in an oven for 30 minutes at 37°C.
4. Lift the sample and hold it still for 30 seconds, weigh the mass of the dressing W2=6.7280 g.
5. The absorption capacity of the dressing is A=(W2-W1)/W1=15.53 g/g.

### Example 7

Measurement of the wet strength of the acylated chitosan wound dressing:
Take a 10x10 cm dressing made from example 5, cut a 2x10cm strip from the dressing, then cut another 2x8 cm strip from the dressing 90 degrees from the previous strip. These two strips are samples for wet strength for the machine direction and cross machine direction.

Place both strips into a Petri-dish containing solution A for 30 seconds, remove the excess solution and test the maximum breaking force of each strip using a tensiometer. These obtain the wet strength of the dressing in machine direction 1.6 N/cm, the cross machine direction 2.3N/cm, the average wet strength of 1.96 N/cm.

### Example 8

Measurement of the bacteriostatic property of the acylated chitosan wound dressing made from example 5:
1. Cut the dressing made from example 5 into 2x2 cm.
2. Place the sample onto a Petri-dish that has been covered with 0.25 ml of 10⁶-10⁷ cfu/g Bacillus subtilis.
3. Place the Petri-dish into an oven at a temperature of 37°C and observe the growth of the bacteria on the plate.

It can be seen that the area underneath the dressing does not show signs of bacterial growth whilst other areas display significant growth of the bacteria, as shown in Fig. 2.

### Example 9

Another absorbent acylated chitosan fibrous wound dressing:
1. Preparation of the reaction solution with a concentration of 0.025 g/ml of succinic anhydride: weigh 273 g of succinic anhydride and dissolve it in 10.93 L of ethanol, keep stirring until all succinic anhydride is fully dissolved.
2. Weigh 500 g of chitosan fibre (degree of deacetylation of 88%, fibre linear density was 1.5 dtex). This will give a molar ratio of anhydride and amino group of chitosan fibre of 1:1. Submerse the fibre in ethanol solution for 30 minutes, then squeeze off all liquid.
3. Place the chitosan fibre into the reaction solution prepared earlier, heat the solution to 50 °C for 100 minutes.
4. Stop the reaction and take the fibre out of solution, squeeze all liquid off the fibre, then wash the fibre with ethanol to remove residue.
5. Place the washed fibre in an ethanol solution containing Tween 20 for 40 minutes, then squeeze off all liquids, dry the fibre before crimping and cutting the fibre into staple fibre (length 60 mm).
6. Measure the degree of substitution of the treated fibre, DS=0.25.
7. Place the fibre through a carding and nonwoven line and convert the fibre in a nonwoven felt with a base weight of 140g/m², cut the felt into 5x5 cm or 10x10 cm dressings and pack them into pouches before putting the packed dressing through a gamma radiation process for sterilisation.

### Example 10

Measurement of the absorption capacity of the acylated chitosan wound dressing (following the method of BS EN 13726-2002).
1. Weigh a 5x5 cm acylated chitosan wound dressing made from the example 9, W1=0.4382g
2. Place the dressing into a Petri-dish containing solution A. The volume of the solution A is 40 times of the weight of the dressing.
3. Place the Petri-dish in an oven for 30 minutes at 37°C.
4. Lift the sample and hold it still for 30 seconds, weigh the mass of the dressing W2=5.3680 g.
5. The absorption capacity of the dressing is A=(W2-W1)/W1=11.25 g/g.

### Example 11

Measurement of the wet strength of the acylated chitosan wound dressing:
Take a 10x10 cm dressing made from example 9, cut a 2x10 cm strip from the dressing, then cut another 2x8 cm strip from the dressing 90 degrees from the previous strip. These two strips are samples for wet strength for the machine direction and cross machine direction.

Place both strips into a Petri-dish containing solution A for 30 seconds, remove the excess solution and test the maximum breaking force of each strip using a tensiometer. This obtains the wet strength of the dressing in machine direction 2.1 N/cm, the cross machine direction 2.9 N/cm, the average wet strength of 2.5 N/cm.

### Example 12

Measurement of the bacteriostatic property of the acylated chitosan wound dressing made from example 9:
1. Cut the dressing made from example 9 into 2x2 cm.
2. Place the sample onto a Petri-dish that has been covered with 0.25 ml of 10⁶-10⁷ cfu/g Pseudomonas aeruginosa bacilli.
3. Place the Petri-dish into an oven at a temperature of 37°C and observe the growth of the bacteria on the plate.

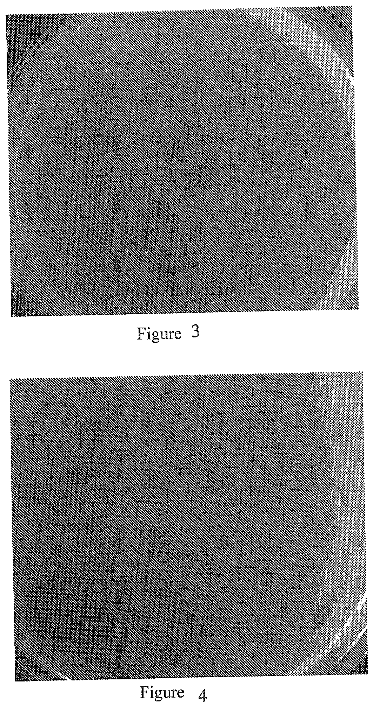

It can be seen that the area underneath the dressing does not show signs of bacterial growth whilst other areas display significant growth of the bacteria, as shown in Fig. 3.

### Example 13

Another absorbent acylated chitosan fibrous wound dressing:
1. Preparation of the reaction solution with a concentration of 0.15 g/ml of succinic anhydride: weigh 2229 g of succinic anhydride and dissolve it in 14.86 L of ethanol, keep stirring until all succinic anhydride is fully dissolved.
2. Weigh 975 g of chitosan fibre (degree of deacetylation of 92%, fibre linear density was 2.2 dtex). This will give a molar ratio of anhydride and amino group of chitosan fibre of 4:1. Submerse the fibre in ethanol solution for 40 minutes, then squeeze off all liquid.
3. Place the chitosan fibre into the reaction solution prepared earlier, heat the solution to 70 °C for 80 minutes.
4. Stop the reaction and take the fibre out of solution, squeeze all liquid off the fibre, then wash the fibre with ethanol to remove residue.
5. Place the washed fibre in an ethanol solution containing Tween 20 for 40 minutes, then squeeze off all liquids, dry the fibre before crimping and cutting the fibre into staple fibre (length 38 mm).
6. Measure the degree of substitution of the treated fibre, DS=0.40.
7. Place the fibre through a carding and nonwoven line and convert the fibre in a nonwoven felt with a base weight of 100g/m², cut the felt into 5x5 cm or 10x10 cm dressings and pack them into pouches before putting the packed dressing through a gamma radiation process for sterilisation.

### Example 14

Measurement of the absorption capacity of the acylated chitosan wound dressing (following the method of BS EN 13726-2002).
1. Weigh a 5x5 cm acylated chitosan wound dressing made from the example 13, W1=0.3758g
2. Place the dressing into a Petri-dish containing solution A. The volume of the solution A is 40 times the weight of the dressing.
3. Place the Petri-dish in an oven for 30 minutes at 37°C.
4. Lift the sample and hold it still for 30 seconds, weigh the mass of the dressing W2=9.5008 g.
5. The absorption capacity of the dressing is A=(W2-W1)/W1=24.4 g/g.

### Example 15

Measurement of the wet strength of the acylated chitosan wound dressing:
Take a 10x10 cm dressing made from example 13, cut a 2x10 cm strip from the dressing, then cut another 2x8 cm strip from the dressing 90 degrees from the previous strip. These two strips are samples for wet strength for the machine direction and cross machine direction.

Place both strips into a Petri-dish containing solution A for 30 seconds, remove the excess solution and test the maximum breaking force of each strip using a tensiometer. These obtain the wet strength of the dressing in machine direction 0.25 N/cm, the cross machine direction 0.60 N/cm, the average wet strength of 0.43 N/cm.

### Example 16

Measurement of the bacteriostatic property of the acylated chitosan wound dressing made from example 13:
1. Cut the dressing made from example 13 into 2x2 cm.
2. Place the sample onto a Petri-dish that has been covered with 0.25 ml of 10⁶-10⁷cfu/g Escherichia coli.
3. Place the Petri-dish into an oven at a temperature of 37°C and observe the growth of the bacteria on the plate.

It can be seen that the area underneath the dressing does not show signs of bacterial growth whilst other areas display significant growth of the bacteria, as shown in Fig. 4.

### Example 17

In order to further enhance the wet strength of the acylated chitosan wound dressing, a small portion of untreated chitosan fibre is blended with acylated chitosan fibre during the dressing making process. This way, the dressing's absorption capacity is largely maintained but the dressing's wet strength has been increased. The method is described as follows:
Chitosan fibre: linear density 1.7 dtex, fibre length 51 mm.

Acylated chitosan fibre: DS=0.35, linear density 2.2 dtex, fibre length 50 mm. The absorbency of solution A of the acylated chitosan fibre is 18 g/g, contain 1% Tween 20.

Manually blend 100 g of chitosan fibre with 1900 g of acylated chitosan fibre, open the fibres with a single cylinder opener then feed the opened fibre into a single cylinder carding machine. The fibre receives further blending during the carding stage and is carded into a uniform web, then needle-punched into a nonwoven with a base weight of 130 gsm.

Cut the fabric into 10x10 cm, pack the dressing into pouches then sterilise the dressing with an EtO process.

The dressing has an absorbency of 12.3 g/g, the dressing's wet strength in machine direction is 0.58 N/cm, in cross machine direction: 0.95 N/cm. The average wet strength of the dressing is 0.77 N/cm.

### Example 18

To make the dressing capable of donating calcium whilst maintaining the properties of the acylated chitosan wound dressing, a small portion of calcium alginate fibre is blended with acylated chitosan fibre during the dressing making process. The method is described as follows:
Calcium alginate: High G, linear density 2.1 dtex, fibre length 50 mm, contains 1% by weight of surfactant.

Acylated chitosan fibre: DS=0.35, linear density 1.7 dtex, fibre length 51 mm. The absorbency of solution A of the acylated chitosan fibre is 18 g/g, containing 1% Tween 20.

Manually blend 200 g of alginate fibre with 1800 g of acylated chitosan fibre, open the fibres with a single cylinder opener then feed the opened fibre into a single cylinder carding machine. The fibre receives further blending during the carding stage and is carded into a uniform web, then needle-punched into a nonwoven fabric with a base weight of 120 gsm.

Cut the fabric into 10x10 cm, pack the dressing into pouches then sterilise the dressing with an EtO process.

The dressing has an absorbency of 14.2 g/g, the dressing's wet strength in machine direction is 0.45 N/cm, in cross machine direction: 0.65 N/cm. The average wet strength of the dressing is 0.55 N/cm. A calcium ion concentration of 30 mg/Kg is measured after submersing the dressing in distilled water.

### Example 19

To further increase the absorbency of the dressing, a small portion of chemically modified cellulose fibre (carboxymethyl cellulose) is blended with acylated chitosan fibre during the dressing making process. The method is described as follows:
Acylated chitosan fibre: DS=0.35, linear density 1.7 dtex, fibre length 51 mm. The absorbency of solution A of the acylated chitosan fibre is 18 g/g, contain 1% Tween 20.

Carboxymethyl cellulose fibre: linear density 1.7 dtex, fibre length 50 mm. The absorbency of solution A is 22 g/g
Manually blend 1000 g of carboxymethyl cellulose fibre with 1000 g of acylated chitosan fibre, open the fibres with a single cylinder opener then feed the opened fibre into a single cylinder carding machine. The fibre receives further blending during the carding stage and is carded into a uniform web, then needle-punched into a nonwoven fabric with a base weight of 140 gsm.

Cut the fabric into 10x10 cm, pack those dressings into pouches then sterilise the dressing with an EtO process.

The dressing has an absorbency of 14.3 g/g, the dressing's wet strength in machine direction is 0.30 N/cm, in cross machine direction: 0.52 N/cm. The average wet strength of the dressing is 0.41 N/cm.

### Example 20

In order to improve the dressing's antimicrobial property, a small portion of antimicrobial fibre is blended with acylated chitosan fibre during the dressing making process. The method is described as follows:
Acylated chitosan fibre: DS=0.35, linear density 1.7 dtex, fibre length 51 mm. The absorbency of solution A of the acylated chitosan fibre is 18 g/g, contain 1% Tween 20.

Silver alginate fibre: High G, linear density 2.2 dtex, fibre length 50 mm. The fibre contains 0.5% by weight of nano silver.

Manually blend 200 g of silver alginate fibre with 1800 g of acylated chitosan fibre, open the fibres with a single cylinder opener then feed the opened fibre into a single cylinder carding machine. The fibre receives further blending during the carding stage and is carded into a uniform web, then needle-punched into a nonwoven fabric with a base weight of 140 gsm.

Cut the fabric into 10x10 cm, pack the dressing into pouches then sterilise the dressing with an EtO process.

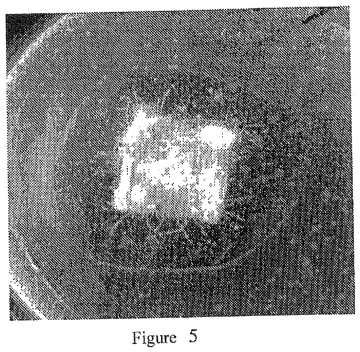

The dressing has an absorbency of 12.1 g/g, the dressing's wet strength in machine direction is 0.46 N/cm, in cross machine direction: 0.60 N/cm. The average wet strength of the dressing is 0.53 N/cm. The antimicrobial property of the dressing is evaluated using the method in example 8. The zone of inhibition of this dressing is shown in Fig. 5.

### Example 21

Another absorbent acylated chitosan fibrous wound dressing is described as follows:
1. Convert 1000 g of chitosan fibre (degree of deacetylation of 90%, fibre linear density was 1.7 dtex, length 51 mm) into a nonwoven fabric with a base weight of 120 gsm. Cut out 1 m² as the sample.
2. Preparation of the reaction solution with a concentration of 0.05 g/ml of succinic anhydride: weigh 205 g of succinic anhydride and dissolve it in 4.5 L of ethanol, keep stirring until all succinic anhydride is fully dissolved.
3. Submerse the above chitosan nonwoven fabric into ethanol for 30 minutes.
4. Place the fabric into the reaction solution prepared in step 2; heat the solution to 60 °C for 75 minutes.
5. Stop the reaction and take the fabric out of solution, squeeze off all liquid, then wash the fabric with ethanol to remove residue.
6. Place the washed fabric in an ethanol solution containing Tween 20 for 30 minutes, then squeeze off all liquids and dry the nonwoven fabric.
7. Measure the degree of substitution of the treated fabric, DS=0.32.
8. Cut the felt into 5x5 cm or 10x10 cm dressings and pack them into pouches before putting the packed dressing through an EtO sterilisation process.

The dressing has an absorbency of 12.9 g/g, wet strength in machine direction of 1.1 N/cm, cross machine direction of 1.4 N/cm, average wet strength of 1.25 N/cm.

### Example 22

To further increase the wet strength of the acylated chitosan wound dressing, a small portion of textile viscose fibre is blended with acylated chitosan fibre during the dressing making process. The method is described as follows:

Acylated chitosan fibre: DS=0.35, linear density 1.7 dtex, fibre length 51 mm. The absorbency of solution A of the acylated chitosan fibre is 18 g/g, contain 0.2% oil 602.

Viscose fibre: linear density 1.5 dtex, fibre length 38 mm, fibre moisture content 11%.

Manually blend 100 g of viscose fibre with 900 g of acylated chitosan fibre, open the fibres with a single cylinder opener then feed the opened fibre into a single cylinder carding machine. The fibre receives further blending during the carding stage and is carded into a uniform web, then needle-punched into a nonwoven fabric with a base weight of 130 gsm.

Cut the fabric into 10x10 cm, pack them into pouches and then sterilise the dressing with an EtO process.

The dressing has an absorbency of 12.3 g/g, the dressing's wet strength in machine direction is 0.7 N/cm, in cross machine direction: 1.5 N/cm. The average wet strength of the dressing is 1.1 N/cm.

### Example 23

In order to further increase the wet strength of the acylated chitosan wound dressing, a larger percentage of untreated chitosan fibre is blended with acylated chitosan fibre during the dressing making process. The method is described as follows:
Chitosan fibre: linear density 1.7 dtex, fibre length 51 mm.

Acylated chitosan fibre: DS=0.35, linear density 2.2 dtex, fibre length 50 mm. The absorbency of solution A of the acylated chitosan fibre is 18 g/g, contain 1% Tween 20.

Manually blend 1900 g of chitosan fibre with 100 g of acylated chitosan fibre, open the fibres with a single cylinder opener then feed the opened fibre into a single cylinder carding machine. The fibre receives further blending during the carding stage and is carded into a uniform web, then needle-punched into a nonwoven fabric with a base weight of 175 gsm.

Cut the fabric into 10x10 cm, pack the dressing into pouches and then sterilise the dressing with an EtO process.

The dressing has an absorbency of 6.9 g/g, the dressing's wet strength in machine direction is 4.7 N/cm, in cross machine direction: 7.0 N/cm. The average wet strength of the dressing is 5.9 N/cm.

## Claims

1. A method for the preparation of a wound dressing comprising acylated chitosan fibre in which the acylated chitosan fibre has a degree of substitution of 0.10-0.50 and wherein the dressing has a minimum wet strength of 0.3 N/cm, the method comprising:
preparing the acylated chitosan fibre by reacting chitosan fibre with an anhydride in a solvent; and making an acylated chitosan dressing through a weaving, knitting or nonwoven process, and then through cutting, packing and sterilizing processes;
or preparing a standard chitosan fabric through a textile process such as weaving, knitting or nonwoven; and chemically modifying the said fabric through an acylation process comprising reacting the chitosan fibre with an anhydride in a solvent, and then through cutting, packing and sterilizing processes,
wherein the anhydride is selected from succinic anhydride, glutaric anhydride, maleic anhydride, phthalic anhydride and diglycolic anhydride, and wherein the solvent is ethanol.

2. A method according to claim 1 **characterized in that** the dressing has an absorbency of 5-25g/g of a solution containing 8.298g/l of sodium chloride and 0.368 g/l of calcium chloride dehydrate.

3. A method according to claim 1 **characterized in that** the molar ratio between the anhydride and the amino group of the chitosan fibre is equal to or greater than 1:1, preferably greater than 2:1, more preferably greater than 4:1.

4. A method according to claim 1 **characterized in that** the fibre linear density of acylated chitosan fibre is between 0.5 dtex and 5 dtex.

5. A method according to any of claim 1 **characterized in that** the fibre length of acylated chitosan fibre is between 3 and 125mm, preferably 25-85 mm.

6. A method according to any of claim 1 **characterized in that** the acylated chitosan fibre contains surfactant or oil as a process aid and/or antimicrobial agents.

7. A method according to any preceding claim wherein the acylation process is performed with a concentration of anhydride in ethanol of 0.1 g/ml - 0.2 g/ml at a temperature of between 30° to 100°C, for between 10 and 120 minutes.

8. A method according to any preceding claim wherein the resultant acylated chitosan fibre is washed with ethanol, followed by drying.

9. A method according to any preceding claim wherein an antimicrobial agent is added to the resultant acylated chitosan fibre.

10. A method according to any preceding claim wherein the acylated chitosan fibre is blended with non-acylated fibres, modified cellulose fibres or other textile fibres.

## Patentansprüche

1. Verfahren zur Herstellung eines Wundverbands, umfassend acylierte Chitosanfaser, in dem die acylierte Chitosanfaser einen Substitutionsgrad von 0.10-0.50 hat, und wobei der Verband eine minimale Nassfestigkeit von 0.3 N/cm hat, wobei das Verfahren umfasst:
Herstellen der acylierten Chitosanfaser durch Reagieren von Chitosanfaser mit einem Anhydrid in einem Lösungsmittel; und Herstellen eines acylierten Chitosanverbands durch einen Web-, Strick- oder Faservliesprozess, und dann durch Schneid-, Verpackungs- und Sterilisierungsprozesse;
oder Herstellen eines Standard-Chitosangewebes durch einen Textilprozess wie zum Beispiel einen Web-, Strick- oder Faservliesprozess; und chemisches Modifizieren des Gewebes durch einen Acylierungsprozess, umfassend ein Reagieren der Chitosanfaser mit einem Anhydrid in einem Lösungsmittel, und dann durch Schneid-, Verpackungs- und Sterilisierungsprozesse,
wobei das Anhydrid ausgewählt ist aus Bernsteinanhydrid, Glutarsäureanhydrid, Maleinsäureanhydrid, Phthalsäureanhydrid und Diglycolanhydrid, und wobei das Lösungsmittel Ethanol ist.

2. Verfahren nach Anspruch 1, **dadurch gekennzeichnet, dass** der Verband ein Absorptionsvermögen von 5-25 g/g einer Lösung hat, die 8.298 g/l Natriumchlorid und 0.368 g/l Calziumchloriddehydrat enthält.

3. Verfahren nach Anspruch 1, **dadurch gekennzeichnet, dass** das molare Verhältnis zwischen dem Anhydrid und der Aminogruppe der Chitosanfaser größer oder gleich 1:1 ist, vorzugsweise größer als 2:1, weiter bevorzugt größer als 4:1.

4. Verfahren nach Anspruch 1, **dadurch gekennzeichnet, dass** die lineare Faserdichte von acylierter Chitosanfaser zwischen 0.5 dtex und 5 dtex ist.

5. Verfahren nach Anspruch 1, **dadurch gekennzeichnet, dass** die Faserlänge der acylierten Chitosanfaser zwischen 3 und 125 mm ist, vorzugsweise 25-85 mm.

6. Verfahren nach Anspruch 1, **dadurch gekennzeichnet, dass** die acylierte Chitosanfaser einen oberflächenaktiven Stoff oder Öl als ein Prozesshilfsmittel und/oder antimikrobielles Mittel enthält.

7. Verfahren nach einem der vorhergehenden Ansprüche, wobei der Acylierungsprozess durchgeführt wird mit einer Konzentration von Anhydrid in Ethanol von 0.1 g/ml - 0.2 g/ml bei einer Temperatur von zwischen 30° bis 100°C während zwischen 10 und 120 Minuten.

8. Verfahren nach einem der vorhergehenden Ansprüche, wobei die resultierende acylierte Chitosanfaser mit Ethanol gewaschen wird, gefolgt von einer Trocknung.

9. Verfahren nach einem der vorhergehenden Ansprüche, wobei ein antimikrobielles Mittel zu der resultierenden acylierten Chitosanfaser hinzugefügt wird.

10. Verfahren nach einem der vorhergehenden Ansprüche, wobei die acylierte Chitosanfaser mit nicht-acylierten Fasern, modifizierten Zellulosefasern oder anderen Textilfasern gemischt wird.

## Revendications

1. Procédé pour la préparation d'un pansement comprenant une fibre de chitosane acylé dans lequel la fibre de chitosane acylé a un degré de substitution de 0,10 à 0,50 et dans lequel le pansement a une résistance à l'humidité minimale de 0,3 N/cm, ce procédé comprenant :
la préparation de la fibre de chitosane acylé en la faisant réagir avec un anhydride dans un solvant, et la fabrication d'un pansement en chitosane acylé par un processus de tissage, de tricotage ou non tissé, puis par des processus de découpe, d'emballage et de stérilisation,
ou la préparation d'un tissu de chitosane standard par un processus textile tel que le tissage, le tricotage ou un processus non tissé, et la modification chimique dudit tissu par un processus d'acylation comprenant la réaction de la fibre de chitosane avec un anhydride dans un solvant, puis par des processus de découpe, d'emballage et de stérilisation,
dans lequel l'anhydride est choisi parmi l'anhydride succinique, l'anhydride glutarique, l'anhydride maléique, l'anhydride phtalique et l'anhydride diglycolique, et dans lequel le solvant est de l'éthanol.

2. Procédé selon la revendication 1 **caractérisé en ce que** le pansement a une capacité d'absorption de 5 à 25 g/g d'une solution contenant 8,298 g/l de chlorure de sodium et 0,368 g/l de chlorure de calcium déshydraté.

3. Procédé selon la revendication 1 **caractérisé en ce que** le rapport molaire entre l'anhydride et le groupe amine de la fibre de chitosane est égal ou supérieur à 1:1, de préférence supérieur à 2:1 et plus préférablement supérieur à 4:1.

4. Procédé selon la revendication 1 **caractérisé en ce que** la densité linéaire de la fibre de chitosane acylé est entre 0,5 dtex et 5 dtex.

5. Procédé selon la revendication 1 **caractérisé en ce que** la longueur de la fibre de chitosane acylé est entre 3 et 125 mm et de préférence entre 25 et 85 mm.

6. Procédé selon la revendication 1 **caractérisé en ce que** la fibre de chitosane acylé contient un surfactant ou une huile comme adjuvant de processus et/ou agent antimicrobien.

7. Procédé selon l'une quelconque des revendications précédentes dans lequel le processus d'acylation est réalisé avec une concentration d'anhydride dans l'éthanol de 0,1 à 0,2 g/ml à une température de 30 à 100 °C pendant 10 à 120 minutes.

8. Procédé selon l'une quelconque des revendications précédentes dans lequel la fibre de chitosane acylé résultante est lavée avec de l'éthanol, puis séchée.

9. Procédé selon l'une quelconque des revendications précédentes dans lequel un agent antimicrobien est ajouté à la fibre de chitosane acylé résultante.

10. Procédé selon l'une quelconque des revendications précédentes dans lequel la fibre de chitosane acylé est mélangée avec des fibres non acylées, des fibres de cellulose modifiée ou d'autres fibres textiles.
